# EUROPEAN PATENT APPLICATION

(11) **EP 2 813 216 A1**
(43) Date of publication of application: **17.12.2014**
(21) Application number: 13002963.0
(22) Date of filing: 10.06.2013
(51) Int. Cl.: A61K 9/14, A61K 9/16, A61K 31/00

(54) **Stabilized amorphous ticagrelor**

(71) Applicant: Zentiva, a.s., 102 37 Praha 10 (CZ)
(72) Inventor: Davis, Dita, Oldbury, West Midlands B26 OdA (GB); Sedmak, Gregor, 1291 Skofljica (SI); Ridvan, Ludek, 102 00 Praha 10 (CZ); Kral, Vladimir, 120 00 Praha 2 (CZ)
(74) Representative: Jirotkova, Ivana

(57) **Abstract**

Solid solution of ticagrelor molecularly dispersed within a polymer matrix formed by an ionic polymer in its solid state, characterized in that the ionic polymer is selected from hydroxypropyl methyl cellulose, polyvinylpyrrolidone or polyvinyl caprolactam-polyvinyl acetate-polyethyleneglycol.

## Description

### Technical Field

The present invention relates to novel stabilized amorphous forms of ticagrelor (I), methods of preparation as well as their use in pharmaceutical compositions.

### Background Art

The compound (1*S*,2*S*,3*R*,5*S*)-3-[7-[(1*R*,2*S*)-2-(3,4-Difluorophenyl)cyclopropylamino]-5-(propylthio)-3H-[1,2,3]triazolo[4,5-d]pyrimidin-3-yl]-5-(2-hydroxyethoxy)cyclopentane-1,2-diol (ticagrelor) of formula I 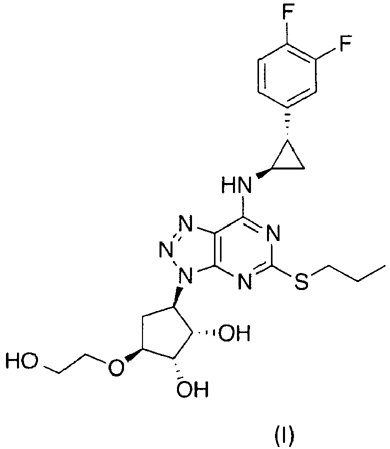 is known to be a platelet aggregation inhibitor that is indicated for the prevention of thrombotic events in patients with myocardial infarction.

Ticagrelor was specifically described in WO 00/34283 displaying also process and use thereof. As described in WO2001092262, Ticagrelor can exist in several different substantially crystalline forms referred to hereafter as polymorphic forms I, II, III and IV. Also amorphous form of ticagrelor was described therein, as well as process of preparation individual forms and use thereof in pharmaceutical formulation. For preparation of pharmaceutical composition however, predominantly crystalline forms of Ticagrelor are used because of lower stability and usually higher content of impurities present in amorphous form.

It was found in WO2011067571 that ticagrelor can form a lot of co-crystals with various coformers such as malonic acid, succinic acid, decanoic acid, salicylic acid, gentisic acid, glutaric acid, vanillin acid etc.

Patent applications WO 2008/024044 and WO 2008/024045 relate to pharmaceuticals formulations with ticagrelor. There are shown compositions with crystalline form II and III and one or more fillers, one or more binders, one or more lubricants and optionally one or more disintegrants.

To pharmaceutical dosage form comprising ticagrelor as pharmaceutically active ingredient, to certain particles of ticagrelor and to processes of preparing the same relates WO2011076749.

As ticagrelor is known to be pharmaceutically active ingredient exhibiting only low solubility, low permeability, and is categorised as a class IV compound under the Biopharmaceutical Classification System (BCS), there is still significant problem to design pharmaceutical composition.

APIs such as Ticagrelor are typically subjected to salt or co-crystal formation so as to improve their solubility and hence bioavailability. One way to increase solubility of ticagrelor is to prepare it in amorphous form.

### Description of the invention

The present invention relates to the solid state structures of (1*S*,2*S*,3*R*,5*S*)-3-[7-[(1*R*,2*S*)-2-(3,4-Difluoro-phenyl)-cyclopropylaminoj-5-(propylthio)-3H-[1,2,3]triazolo[4,5-d]pyrimidin-3-yl]-5-(2-hydroxyethoxy)-cyclopentane-1,2-diol (hereafter referred to as Ticagrelor) in the form of a stable amorph and their preparation and use of the same in pharmaceutical formulations.

Crystalline solid is characterized by its long-range order. On the other hand, amorphous solids lack the long-range order. The molecular arrangement in the amorphous solid may be represented by a frozen liquid with the rheological properties of a solid.

As is known in the art, because of the lack of a long-range order and usually high surface area, amorphous solids typically exhibit higher solubility then crystalline form. In order to increase solubility of a crystalline solid, it is thus beneficial to prepare active pharmaceutical ingredient in an amorphous form.

If a crystalline material is heated until its melting point is reached (Tm), the material is transformed from solid to liquid state. This is reversible, so upon cooling, the molten liquid rearranges back to crystal lattice. If, however, the liquid is cooled sufficiently fast below its melting point, crystallization may be prevented forming a super-cooled liquid. In the super-cooled liquid is cooled enough to reach glass transition temperature (Tg), the molecules are kinetically frozen and the super-cooled liquid solidifies into a glass. In general, molecules in a super-cooled liquid state have much higher mobility as in a glassy state as described in Remington: The Science and Practice of Pharmacy, Pharmaceutical Press, 21nd edition.

Since the molecules even in a glassy state have some mobility, it is known in the art that it is beneficial that the glass transition temperature of active pharmaceutical ingredient is at least 20°C higher, more preferably at least 30 °C higher and most preferably at least 40 °C higher than the actual storage conditions.

Ticagrelor is characterized by its low solubility under the Biopharmaceutics Classification System (BCS). Therefore, there exists a clear need to increase solubility of ticagrelor. One way to increase solubility of ticagrelor is to prepare it in an amorphous form.

Amorphous ticagrelor has relatively low glass transition temperature (Tg), which is about 46 °C. Therefore, the amorphous form undergoes recrystallization upon storage conditions. It is therefore highly beneficial to stabilize amorphous form of ticagrelor by Tg increase in order to prevent recrystallization.

Amorphous form of ticagrelor can be stabilized by mixing ticagrelor with a second component. There are a number of different second components that can be used to stabilize ticagrelor. In general, all second components used to stabilize ticagrelor have a common characteristic which is that they decrease mobility of ticagrelor molecules and thus prevent recrystallization.

One type of a second component used to stabilize amorphous ticagrelor are polymers. Suitable polymers used to stabilize amorphous ticagrelor may be selected from, but not restricted to water soluble as well as water insoluble polymers. Typical water soluble polymers suitable to stabilize amorphous ticagrelor are povidone, copovidone, hypromellose, hydroxypropylcellulose, polyvinyl caprolactam-polyvinyl acetate-polyethylene glycol graft copolymer (Soluplus™), polyvinyl alcohol and similar. Typical water insoluble polymers suitable to stabilize amorphous ticagrelor are methylcellulose, ethylcellulose, polymethacrylates, hypromellose phthalate, hypromellose acetate succinate, cellulose acetate phthalate, carboxymethyl ethyl cellulose and crospovidone.

The API should be immobilised by interaction with polymer for i.e. via hydrogen bonding. Soluplus and PVP K30 polymers have amide structure, which provides hydrogen bond acceptors for the API molecule. Therefore, glass solutions of Ticagrelor are thermodynamically stabilised as their compositions are similar to those of liquid solutions. The total interactions between the API and polymer are stronger than the self-assembly of the API molecules. As a result Ticagrelor molecules do mix completely as one single phase of free molecules dispersed in the polymer-molecularly dispersed within polymer matrix. This results in a single homogeneous amorphous phase of solid solutions.

Second type of a second component to stabilize amorphous ticagrelor are high surface area inorganic oxides. Suitable high surface area inorganic oxides used to stabilize amorphous ticagrelor may be selected from, but not restricted to silicon dioxide, titanium dioxide, zinc oxides, calcium silicate, aluminum oxide, magnesium aluminum silicate, bentonite, attapulgite, kaolin, zeolite and other molecular sieves.

Third type of a second component to stabilize amorphous ticagrelor are carbohydrates. Suitable carbohydrates used to stabilize amorphous ticagrelor may be selected from, but not restricted to lactose, raffinose, sucrose, trehalose, maltose, dextran, maltodextrin and alpha, beta and gamma types of cyclodextrins.

There exist a number of possible processes to prepare a stabilized amorphous form of ticagrelor.

One possible process is the so-called solvent processes. What is common to all solvent processes is that the active ingredient is dissolved in a solvent or in any mixture thereof. Solvents can be water or any organic solvents. Some examples of possible organic solvents are methanol, ethanol, ethyl acetate, isopropanol, acetone, dichloromethane, tetrahydrofuran etc. In the next step, a second component to stabilize ticagrelor is added forming either solution or suspension. The solvent is then quickly removed and a super-cooled glassy solid remains. Some concrete solvent processes used to prepare composition of ticagrelor in stabilized amorphous form may be selected from, but not restricted to removal of a solvent in a rotary evaporator, fluid bed granulation, spray drying, electrospinning, freeze drying and similar.

The second possible process is the process without the use of a solvent. In this process, ticagrelor is mixed directly with the second component to stabilize amorphous form and melted in order to form a melt. In general, the melt is heated to approximately 20-40 °C above the Tg of the melted system in order to decrease the viscosity of a system to such a degree that it is possible to process the system. The melt is subsequently cooled in order to form a super-cooled glassy solid. Some concrete examples of this process may be selected from, but not restricted to hot melt extrusion, hot melt granulation in a high shear mixer, solvent-free melt granulation in a fluid bed granulator and the like.

The API should be immobilised by interaction with polymer for i.e. via hydrogen bonding. Soluplus and PVP K30 polymers have amide structure, which provides hydrogen bond acceptors for the API molecule. Therefore, glass solutions of Ticagrelor are thermodynamically stabilised as their compositions are similar to those of liquid solutions. The total interactions between the API and polymer are stronger than the self-assembly of the API molecules. As a result Ticagrelor molecules do mix completely as one single phase of free molecules dispersed in the polymer-molecularly dispersed within polymer matrix. This results in a single homogeneous amorphous phase of solid solutions.

The invention relates to a solid pharmaceutical composition comprising ticagrelor and at least one second component used to stabilize amorphous ticagrelor.

The composition comprising ticagrelor and at least one second component used to stabilize amorphous ticagrelor typically comprises at least one filler. Water-soluble fillers are generally preferred. Suitable fillers comprising ticagrelor composition may be selected from, but not restricted to monosaccharides, oligosaccharides and sugar alcohols like glucose, fructose, saccharose, lactose monohydrate, lactose anhydrous, raffinose, isomalt, trehalose, dextrates, mannitol, erythritol, sorbitol, maltitol, xylitol and lactitol, compressible sugar, dibasic calcium phosphate dihydrate and mixtures thereof. Preferred fillers are lactose, mannitol, xylitol.

The composition comprising ticagrelor and at least one second component used to stabilize amorphous ticagrelor typically comprises binder. Suitable binders may include povidone, copovidone, cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, maltose, starch, pregelatinized starch, polymethacrylates and mixtures thereof. Hydroxypropylcellulose and maltose are particularly preferred. The composition comprising ticagrelor preferably comprises 0.5 to 30 wt.-% of binder, more preferably 1 to 7 wt.-% of binder, most preferably 2 to 4 wt.-% of binder.

The composition comprising ticagrelor and at least one second component used to stabilize amorphous ticagrelor may optionally comprise surfactants. Suitable surfactants may be selected from anionic, cationic, ampholytic and non-ionic surfactants such as sodium lauryl sulfate, cetrimide, N-dodecyl-N,N-dimethylbetaine, polysorbates (such as Tweens^{®}), poloxamers and mixtures thereof. Preferred is sodium lauryl sulfate. The composition comprising ticagrelor preferably comprises 0.1 to 4.0 wt.-% of surfactant, most preferably 0.5 to 2.0 wt.-% of surfactant.

The composition comprising ticagrelor and at least one second component used to stabilize amorphous ticagrelor typically comprises lubricant and/or glidant. Suitable lubricants and/or glidants may be selected from magnesium stearate, hydrogenated vegetable oil, hydrogenated castor oil, talc, sodium stearyl fumarate, colloidal silicon dioxide, magnesium trisilicate and mixtures thereof. Stearic acid, magnesium stearate and sodium stearyl fumarate and colloidal silicon dioxide are particularly preferred.

The tablet and/or capsule comprising ticagrelor and at least one second component used to stabilize amorphous ticagrelor can optionally be coated by any conventional coating. Suitable coating agent may be selected from methylcellulose, hydroxypropylmethyl cellulose, hydroxypropylcellulose, acrylic polymers, ethylcellulose, cellulose acetate phthalate, polyvinyl acetate phthalate, hydroxypropyl methylcellulose phthalate, polyvinylalcohol, sodium carboxymethylcellulose, cellulose acetate, cellulose acetate phthalate, gelatin, methacrylic acid copolymer, polyethylene glycol, shellac, sucrose, titanium dioxide and carnauba wax. Preferred are polyethylene glycol, hydroxypropylmethyl cellulose and polyvinylalcohol.

### Brief Description of Drawings

Figure 1 shows a powder X-ray diffraction pattern of Ticagrelor/HPMC (1:2) solid solution.
Figure 2 shows a powder X-ray diffraction patterns of Ticagrelor/HPC (1:1) (top) and Ticagrelor (bottom).
Figure 3 shows modulated DSC heat flow thermograms of Ticagrelor (a) and of solid solution of Ticagrelor/PVP K30 (1:2) (b).
Figure 4 shows XRPD data Ticagrelor/HPC (1:2) before and after stress testing (before-medium line, after 6 days- top, after 4 weeks -bottom (re-crystallization to form I-Ticagrelor/HPC (1:2)).

### Examples

DSC curves were measured with a Pyris 1 (Perkin Elmer) device. The sample charge was 3-4 mg, heating rate 10 °C/min. Nitrogen was used as a carrier gas (20 ml/min).

### Example 1 -Electrospinning - tablets

| | 1A | 1B | 1C | 1D |
|---|---|---|---|---|
| Ticagrelor | 90.00 | 90.00 | 90.00 | 90.00 |
| Povidone | 90.00 | 180.00 | | |
| Soluplus | | | 90.00 | 180.00 |
| Mannitol | 117.00 | | | 27.00 |
| Lactose monohydrate | | 27.00 | 117.00 | |
| Magnesium stearate | 3.00 | | 3.00 | |
| Sodium stearyl fumarate | | 3.00 | | 3.00 |
| Total (mg) | 300.00 | 300.00 | 300.00 | 300.00 |

Ticagrelor and either povidone or soluplus are dissolved in ethanol. Solution is electrospun in order to obtain amorphous ticagrelor together with a second component in order to stabilize amorphous ticagrelor. Electrospun material is subsequently mixed with either mannitol or lactose and magnesium stearate or sodium stearyl fumarate and encapsulated into a hard gelatine capsule.

### Example 2- Electrospinning - capsules

| | 2A | 2B | 2C |
|---|---|---|---|
| Ticagrelor | 90.00 | 90.00 | 90.00 |
| Povidone | 150.00 | | |
| Soluplus | | 225.00 | 150.00 |
| Total(mg) | 240.00 | 315.00 | 240.00 |

Ticagrelor and either povidone or soluplus are dissolved in ethanol. Solution is electrospun in order to obtain amorphous ticagrelor together with a second component in order to stabilize amorphous ticagrelor. Electrospun material is inserted into a standard hard gelatine capsule of size 0 elongated.

### Example 3 - Spray drying

Ticagrelor and either povidone or silicon dioxide or hypromellose or soluplus or hypromellose acetate succinate or methacrylic acid copolymer type A or maltodextrin or beta cyclodextrin are mixed with methanol or methanol/water mixture in order to get a solution or a suspension. Solution or suspension is spray dried in order to obtain amorphous ticagrelor together with a second component in order to stabilize amorphous ticagrelor. Spray dried material is subsequently mixed with mannitol and either magnesium stearate or sodium stearyl fumarate and encapsulated into a hard gelatine capsule.

| | 3A | 3B | 3C | 3D | 3E | 3F | 3G | 3H |
|---|---|---|---|---|---|---|---|---|
| Ticagrelor | 90.00 | 90.00 | 90.00 | 90.00 | 90.00 | 90.00 | 90.00 | 90.00 |
| Povidone | 180.00 | | | | | | | |
| Silicon dioxide | | 180.00 | | | | | | |
| Hypromellose | | | 180.00 | | | | | |
| Soluplus | | | | 180.00 | | | | |
| Hypromellose acetate succinate | | | | | 180.00 | | | |
| Methacrylic acid copolymer type A | | | | | | 180.00 | | |
| Maltodextrin | | | | | | | 180.00 | |
| Beta cyclodextrin | | | | | | | | 180.00 |
| Mannitol | 127.00 | 127.00 | 127.00 | 127.00 | 127.00 | 127.00 | 127.00 | 127.00 |
| Magnesium stearate | 3.00 | | 3.00 | | 3.00 | | 3.00 | |
| Sodium stearyl fumarate | | 3.00 | | 3.00 | | 3.00 | | 3.00 |
| Total(mg) | 400.00 | 400.00 | 400.00 | 400.00 | 400.00 | 400.00 | 400.00 | 400.00 |

### Example 4 - Preparation of glass solutions by quick and slow evaporation technique

a) API and polymer (HPMC 6cP, PVP K30 and Soluplus) (1:1 w/w, 100 mg) were dissolved in 2.5 ml of DCM(75%) : methanol. The solvent mixture was removed under reduced pressure yielding the glass solution.
b) API and polymer (HPMC 6cP, HPC, PVP and Soluplus) (1:2 w/w, 100 mg) were dissolved in 4 ml of DCM(75%): methanol. The solvent mixture was removed under reduced pressure, yielding the glass solution.

In the case of slow evaporation the solvent was allowed to evaporate at the RT overnight.

The products were characterized by XRPD and DSC analysis.

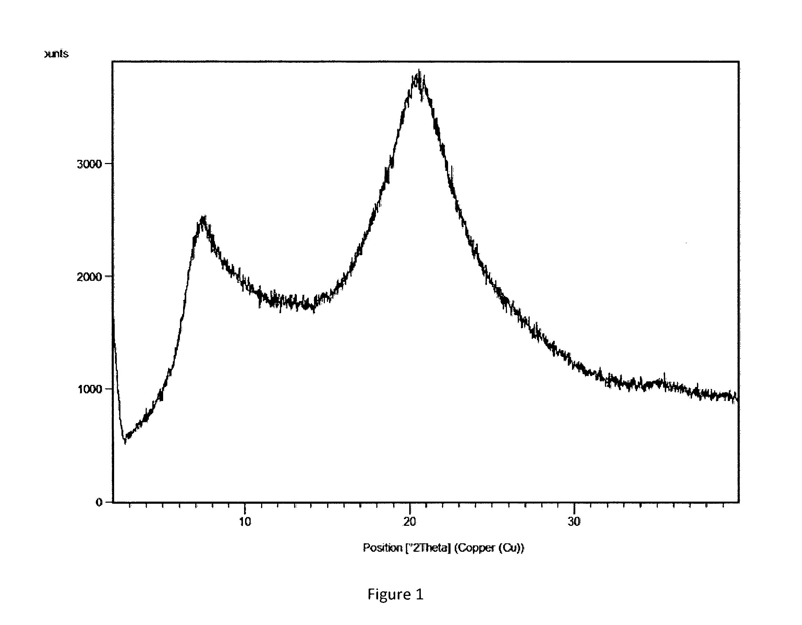

In X-ray powder diffraction (XRPD) analysis, the peak patterns for the glass solutions displayed two broad peaks (amorphous halo) as shown in Fig. 1 as a representative example.

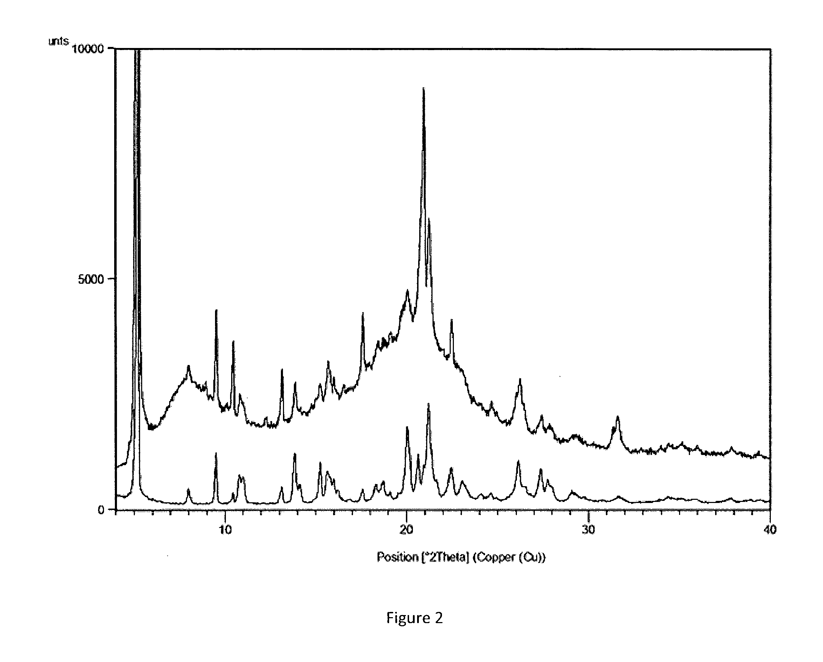

However, the solid solution comprising the HPC polymer in 1:1 ratio showed a crystalline pattern corresponding to the Form I of Ticagrelor as demonstrated in Figure 2.

In differential scanning calorimetry (DCS) measurements, the solid solutions exhibited a single glass transition temperature (Tg) indicating complete miscibility between the API and polymer. In all cases the Tg values were higher for solid solution of 1:2 ratio than those for 1:1 ratio. Therefore, the 1:2 API to polymer ratio was able to form more physically stable solid solutions. It is known that the physical stability of the solid solution is increased with a higher glass transition temperature (Hancock and Zografi, 1997).

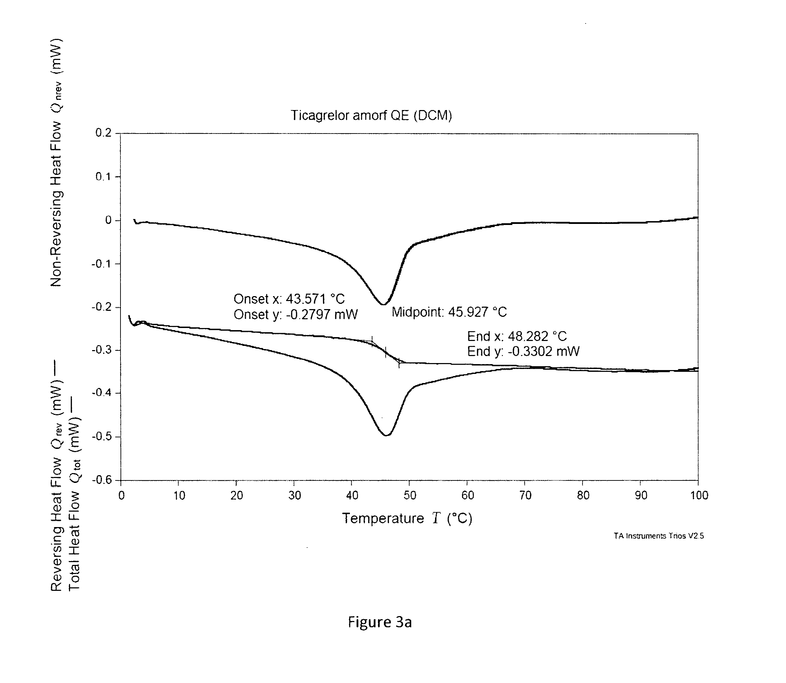

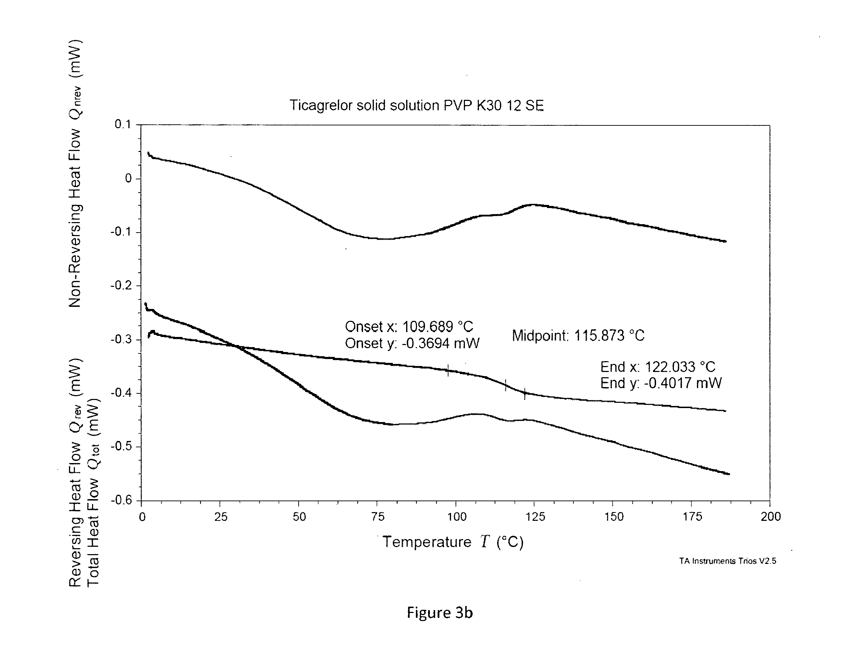

Comparing all recorded Tg values, the highest glass transition temperature (Tg 116°C) was observed for the PVP K30 (1:2) solid solution (Figure 3). The highest glass transition temperature for HPMC (1:2) solid solution was observed Tg 72 °C and for Soluplus (1:2) solid solution Tg was 64 °C.

Ticagrelor is a crystalline drug with a melting point at about 46 °C, while PVP K30 is amorphous solid with Tg value of 167 °C. Therefore the observed single Tg value of 116 °C for this solid matrix is the indication that the solid solution is consisted of only one phase.

### Example 5 - Physical stability testing

Stress tests of the products were carried out at controlled temperature (50 °C) for 6 days and then further for 4 weeks without measuring the relative humidity. The changes in amorphous solid solution samples were analyzed by XRPD.

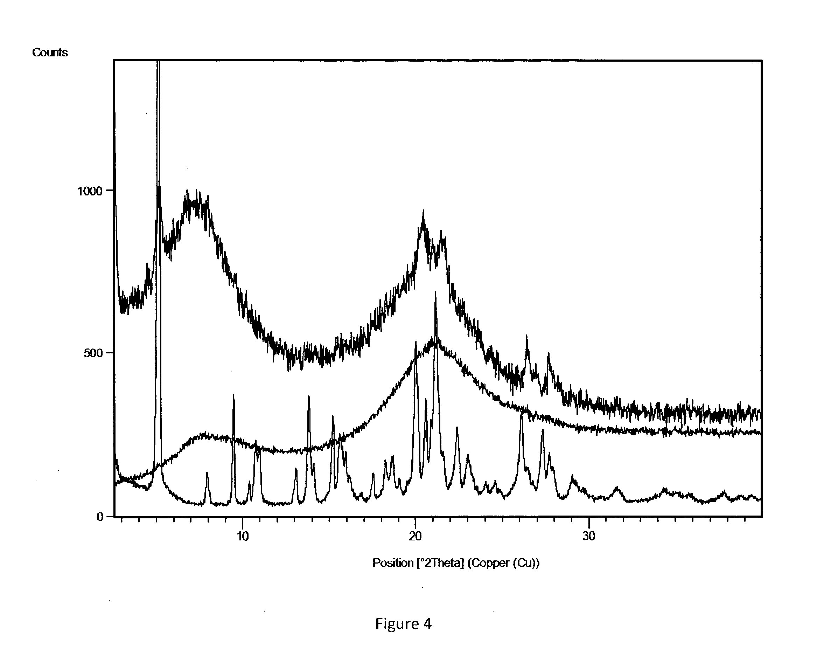

From the XRPD results it was found out that the only re-crystallisation of amorphous API to Form I was observed with HPC polymer in 1:2 ratio. The XRPD data before and after stress testing are displayed in Fig. 4. The rest of the glass solutions remained in amorphous form after 4 weeks of stability testing.

### Example 6 - Hot melting extrusion

All solid solutions were prepared at an API/polymer ratio of 1:1 and 1:2.5 (w/w) respectively, using the hot melting extrusion technique. Ticagrelor and polymer blends were extruded using a twin-screw extruder with a temperature cascade rising to 160 °C.

Melt extrusion resulted in amorphous solid solutions as assessed by XRPD and DSC for all three polymers (PVP K30, Soluplus, HPMC) in ratios from 1:1 to 1:2.5 ratio respectively.

DSC thermograms displayed a single Tg for all compounds confirming that extrusion resulted in a one phase amorphous products. The Tg values obtained from the melt extruded samples were similar to those of the quick and slow evaporation products. The highest Tg value of the Ticagrelor/PVP K30 (1:2 w/w) melt extrudate was found to be around 2 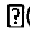 C lower than that of obtained by the quick evaporation technique.

XRPD spectra of all solid solutions exhibited also evidence of amorphous solid solutions (amorphous halo). The data are consistent with the XRPD results of the solid solutions prepared by quick and slow evaporation technique.

## Claims

1. Solid solution of ticagrelor molecularly dispersed within a polymer matrix formed by an ionic polymer in its solid state, **characterized in that** the ionic polymer is selected from hydroxypropyl methyl cellulose, polyvinylpyrrolidone or polyvinyl caprolactam-polyvinyl acetate-polyethyleneglycol.

2. Amorphous solid solution of ticagrelor molecularly dispersed within hydroxylpropyl methyl cellulose, having an X-ray diffraction pattern as shown in Fig. 1.

3. Amorphous solid solution of ticagrelor according to claim 1, **characterized in that** it is a single-phase with a glass transition temperature (Tg) of more than 50°C.

4. Amorphous solid solution of ticagrelor molecularly dispersed within polyvinylpyrrolidone, **characterized in that** it is a single-phase with a glass transition temperature (Tg) of more than 50°C.

5. Amorphous solid solution of ticagrelor molecularly dispersed within polyvinyl caprolactam-polyvinyl acetate-polyethyleneglycol, **characterized in that** it is a single-phase with a glass transition temperature (Tg) of more than 50°C.

6. A solid solution according to any of the preceding claims, wherein the ratio of the amount by weight of ticagrelor within the solid solution to the amount by weight of the ionic polymer is from 1:1 to 1:2.5.

7. A process for the preparation of amorphous solid solution of ticagrelor according to claim 1 using a solvent comprising quick or slow evaporation, **characterized in that** the solvent is a mixture of DCM and methanol.

8. A solid pharmaceutical composition comprising solid solution of ticagrelor molecularly dispersed within a polymer matrix formed by an ionic polymer in its solid state, wherein the ionic polymer is selected from hydroxypropyl methyl cellulose, polyvinylpyrrolidone or polyvinyl caprolactam-polyvinyl acetate-polyethyleneglycol, **characterized in that** it further comprises at least one filler, at least one binder, optionally a surfactant, at least one lubricant and/or glidant, and optionally is coated.

9. The solid pharmaceutical composition according to claim 8, **characterized in that** the filler is selected from glucose, fructose, saccharose, lactose monohydrate, lactose anhydrous, raffinose, isomalt, trehalose, dextrates, mannitol, erythritol, sorbitol, maltitol, xylitol and lactitol, compressible sugar, dibasic calcium phosphate dihydrate and mixtures thereof, wherein preferred fillers are lactose, mannitol and xylitol.

10. The solid pharmaceutical composition according to claim 8, **characterized in that** the binder is selected from povidone, copovidone, cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, maltose, starch, pregelatinized starch, polymethacrylates and mixtures thereof, wherein hydroxypropylcellulose and maltose are particularly preferred.

11. The solid pharmaceutical composition according to claim 8, **characterized in that** it optionally comprises at least one surfactant selected from sodium lauryl sulfate, cetrimide, N-dodecyl-N,N-dimethylbetaine, polysorbates, poloxamers and mixtures thereof, wherein preferred is sodium lauryl sulfate.

12. The solid pharmaceutical composition according to claim 8, **characterized in that** the lubricant and/or glidant is selected from stearic acid, magnesium stearate, hydrogenated vegetable oil, hydrogenated castor oil, talc, sodium stearyl fumarate, colloidal silicon dioxide, magnesium trisilicate and mixtures thereof, wherein stearic acid, magnesium stearate, sodium stearyl fumarate and colloidal silicon dioxide are particularly preferred.

13. The solid pharmaceutical composition according to claim 8, coated using a coating agent selected from methylcellulose, hydroxypropyl methylcellulose, hydroxypropylcellulose, acrylic polymers, ethylcellulose, cellulose acetate phthalate, polyvinyl acetate phthalate, hydroxypropyl methylcellulose phthalate, polyvinylalcohol, sodium carboxymethylcellulose, cellulose acetate, cellulose acetate phthalate, gelatin, methacrylic acid copolymer, polyethylene glycol, shellac, sucrose, titanium dioxide and carnauba wax, wherein preferred are polyethylene glycol, hydroxypropyl methylcellulose and polyvinylalcohol.

14. The solid pharmaceutical composition comprising solid solution of ticagrelor molecularly dispersed within polyvinylpyrrolidone, wherein the ratio of the amount by weight of ticagrelor within the solid solution to the amount by weight of the ionic polymer therein is from 1:1 to 1:2.5, **characterized in that** mannitol and magnesium stearate are used as filler and lubricant, resp.

15. The solid pharmaceutical composition comprising solid solution of ticagrelor molecularly dispersed within hydroxylpropyl methyl cellulose, wherein the ratio of the amount by weight of ticagrelor within the solid solution to the amount by weight of the ionic polymer therein is from 1:1 to 1:2.5, **characterized in that** mannitol and magnesium stearate are used as filler and lubricant, resp.
